Europäisches Patentamt

European Patent Office

Office européen des brevets

(11) Publication number: **0 156 565 B1**

(12) **EUROPEAN PATENT SPECIFICATION**

(45) Date of publication of patent specification: **31.07.91**  (51) Int. Cl.⁵: **A61L  15/16**

(21) Application number: **85301597.2**

(22) Date of filing: **07.03.85**

(54) Antipruritic compositions and plasters.

(30) Priority: **07.03.84 JP 41994/84**

(43) Date of publication of application:
**02.10.85 Bulletin  85/40**

(45) Publication of the grant of the patent:
**31.07.91 Bulletin  91/31**

(84) Designated Contracting States:
**DE FR GB IT**

(56) References cited:
**GB-A- 2 045 618**
**GB-A- 2 122 893**
**US-A- 4 307 717**

**PATENTS ABSTRACTS OF JAPAN, vol. 8, no.
114 (C-225)[1551], 26th May 1984; & JP-A-59
27 978 (TERUMO K.K.) 14-02-1984**

(73) Proprietor: **YAMANOUCHI PHARMACEUTICAL
CO. LTD.
No. 3-11 Nihonbashi-Honcho, 2-chome Chuo-
ku
Tokyo(JP)**

(72) Inventor: **Senuma, Makoto
No. 1-32-26-403, Shimura Itabashi-ku
Tokyo(JP)**
Inventor: **Kondoh, Shigeru
No. 764-1-103, Shirakuwa
Urawa-shi Saitama(JP)**
Inventor: **Kawase, Tadamasa
No. 1104-8, Oya hongo
Ageo-shi Saitama(JP)**
Inventor: **Nakagawa, Yoshihiko
No. 369-18,Mukoyama
Ageo-shi Saitama(JP)**

(74) Representative: **Geering, Keith Edwin et al
REDDIE & GROSE 16 Theobalds Road
London WC1X 8PL(GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

EP 0 156 565 B1

## Description

This invention relates to antipruritic plasters containing glycyrrhetinic acids as essential medical component.

Glycyrrhetinic acid is the hydrolyzed product of glycyrrhizin (glycyrrhizic acid) which is an extract component of Glycyrrhizae Radix; it possesses antiphlogistic activity and antipruritic activity,and is frequently used as therapeutic agent for eczema, dermatitis neurotica, and pruritus cutanea.

Glycyrrhetinic acid itself is easily soluble in pyridine and somewhat soluble in ethanol and chloroform but is almost insoluble in fats and oils, hydrocarbons, and water, which makes satisfactory preparations thereof difficult to achieve.

In the treatment of pruritus cutanea such as skin disease and insect bites it is necessary to select a form of preparation suitable for the cause of the itching, various skin symptoms etc. However, in solutions, the solvent is evaporated off by the body heat to deposit crystals, whereby the medical effect obtained,and also its durability, are insufficient. On the other hand, in ointments, etc., the deposition of crystals of medicament is unavoidable when the medical component has poor compatibility with the base, and hence the medical effect and its durability are again poor; an ointment also has the defect that it stains clothes due to its stickiness. A plaster may be considered as a form of preparation having no such defects but such a product is not known for the antipruritic agents used in this invention.

It is known that glycyrrhetinic acid is soluble is crotamiton and fatty acid dialkylamides such as capric acid N,N-dimethylamide, etc., (Japanese Patent Publication Nos. 25,404/'67 and 2,5406/'67),but as preparations of glycyrrhetinic acid, there are presently known only a solution of glycyrrhetinic acid in a volatile solvent such as ethanol, etc., an ointment mainly composed of the acid and polyethylene glycol, and a hydrophilic ointment mainly composed of the acid and a carboxyvinyl polymer (see, for example, Japanese Patent Publication Nos. 25,405/'67 and 25,406/'67; Japanese Patent Publication (OPI) No. 47,520/'74 - "OPI" indicating herein an unexamined published patent application open to public inspection).

For glycyrrhetinic acid esters and salts thereof having improved water or oil solubility, various forms of preparation are known (Japanese Patent Publication (OPI) Nos. 149,222/'82 and 27,815/'84) but the form of a plaster is not known.

The percutaneous administration of various medicaments, in particular, steroid hormones, nonsteroidal antiflammatory agents, antibacterials, antitumor agents, etc., has hitherto been attempted. For example, U. S. Patent No. 4,307,717 and European Patent No. 72,251 describe antipruritic agents such as benzoin, calamine, camphor, menthol, phenol, sulfur, etc., and suggest that these antipruritic agents can be used in the form of a flexible liquid-absorbent or an adhesive bandage for antipruritic purposes, but the techniques disclosed therein cannot be applied to water-insoluble glycyrrhetinic acid.

Japanese Patent Publication (OPI) Nos. 15,861/'83; 105,915/'83 and 36,608/'84 describe that glycyrrhetinic acid and similar materials can be widely used as medicaments in specific bases for medicines for external application, but there is no description of practical preparations and the techniques described cannot be applied as they are to glycyrrhetinic acid which is insoluble in water and scarecely soluble in fats and oils.

Previously, plasters using glycyrrhetinic acid and related compounds as antipruritics have not been known. GB-A-2045618 discloses a plaster in which medicament (not glycyrrhetinic acid or related compound) is in a base comprising thermoplastic elastomer, oil or higher fatty acid, and tackifying resin (e.g.rosin). JP-A-5927978 describes use in an adhesive tape of a minor amount of glycyrrhetinic acid in ethanol solution as penetration assistant for the analgesic and anti-inflammatory dry indometacin in an acrylic resin binder.

From investigations on the compatibility of glycyrrhetinic acid with various bases or other medicaments for developing antipruritic plasters containing glycyrrhetinic acid as the antipruritic medical component, we have found that an ordinary ointment base such as a carboxyvinyl polymer has poor affinity for plaster and is difficult to use as it is for the preparation of plaster.

On the other hand we have found that crotamiton,which is known to have good compatibility with glycyrrhetinic acid (but was of unknown affinity for plaster base and had never been used for plasters) shows good affinity for plasters using rubber compound adhesive plaster bases or acrylic resins.

Furthermore, we have discovered that glycyrrhetinic acid is soluble not only in crotamiton but also in benzyl alcohol, phenethyl alcohol, diphenhydramine, and chlorpheniramine at a solubility higher than 10%, and in diethylene glycol monoethyl ether at a solubility higher than 6%, and that when such solution is compounded with a plaster base, the medicament can show good compatibility without deposition of crystals thereof.

Since benzyl alcohol, phenethyl alcohol, diphenhydramine, and chlorpheniramine not only increase the

2

affinity of glycyrrhetinic acid with plaster base but also have local anesthetic and antipruritic actions themselves, they have great merit as compounding agents for antipruritic agents.

The present invention has been accomplished based on the new knowledge about the compatibility of three components - glycyrrhetinic acid, solvent therefor, and plaster base - leading to the provision for the first time of a glycyrrhetinic acid plaster.

It has also been found that this new knowledge applies also to glycyrrhizin and monoammonium glycyrrhizinate which show the same medical effect as glycyrrhetinic acid and are insoluble in water, fats, oils, and hydrocarbons,and to dipotassium glycyrrhizinate which has the same medical effect as glycyrrhetinic acid and shows an improved solubility in water but also inferior solubility in fats and oils and hydrocarbons.

Where the context allows, the term "glycyrrhetinic acids" herein is to be understood as including not only glycyrrhetinic acid but also glycyrrhizin, and salts of glycyrrhrizin.

The invention provides an antipruritic plaster comprising a backing having formed on one surface a pressure-sensitive layer containing

(1) at least one medicament selected from glycyrrhetinic acid glycyrrhizin and salts of glycyrrhizin;

(2) at least one solvent for (1) selected from benzyl alcohol, phenethyl alcohol, diphenhydramine, chlorpheniramine, and crotamiton;

(3) a base comprising (a) or (b)

(a) at least one rubber compound adhesive plaster base selected from natural rubber, styrene-butadiene copolymer elastomer, and styrene-isoprene-styrene copolymer elastomer; at least one tackifying agent selected from petroleum resin, rosin, hydrogenated rosin, and ester gum; and at least one softener selected from polybutene, liquid paraffin, higher fatty acid esters, silicone oils and vegetable oils,

or

(b) acrylic resin.

The invention also provides an adhesive medicament mass of components (1), (2) and (3) above.

The antipruritic plaster of this invention can be a self-adhesive plaster prepared by coating a backing such as flannel, plastics film, nonwoven fabric, etc., with an adhesive mass containing the aforesaid necessary components and can take various forms such as sheet, film, tape, patch, etc.

The antipruritic plaster of this invention may contain glycyrrhetinic acids as at least one antiflammatory and antipruritic component and can include other antipruritic component(s) and/or other medical effect component(s).

Among the solvents for dissolving glycyrrhetinic acids, benzyl alcohol and phenethyl alcohol possess both a local anesthetic and an antipruritic action, and further the antihistamine agents such as diphenhydramine, chlorpheniramine, etc., and crotamiton possess an anti-pruritic action, and hence they can be used with advantage in the present invention.

Amongst other medicaments which may be compounded in the antipruritic plasters of this invention in addition to the glycyrrhetinic acids there are those which do not dissolve the latter but do not hinder their compatibility with the plaster base and solvent. Examples are antihistamic salts of diphenhydramine (e. g., diphenhydramine hydrochloride), salts of chlorpheniramine (e. g., chlorpheniramine maleate), etc.; antibacterials such as chlorhexidine gluconate, chlorhexidine dihydrochloride, dequalinium chloride, isopropylmethylphenol, benzalkonium chloride, benzethonium chloride, etc.; and odorants such as menthol, etc., which do not possess an antipruritic effect but may be desired.

A suitable compounding amount of glycyrrhetinic acids for sufficient anti-inflamatory and antipruritic action is e.g. 0.5 to 10%, preferably 1 to 4%, by weight of the total medicament-containing adhesive mass when the latter contains no other medicament or solvent having medicinal action; and is e.g. 0.1 to 5%, preferably 0.2 to 2%, by weight of said total mass when the latter does contain other medicament and/or solvent having medicinal action.

The amount of solvent (2) used is suitably 3 to 20 parts by weight per 1 part by weight of glycyrrhetinic acid (for dissolving); it may be e.g. 1 to 20%, preferably 5 to 15%, by weight of the total medicament-containing adhesive mass.

For producing the anti-pruritic plaster of this invention, glycyrrhetinic acids (and optionally other medical effect component or components) can be dissolved in at least one solvent selected from benzyl alcohol, phenethyl alcohol, diphenhydramine, chlorpheniramine and crotamiton, (optionally with co-solvent N-methyl-2-pyrrolidone, lauric acid diethanolamide or diethylene glycol monoethyl ether) and the solution uniformly kneaded with the plaster base to form a medicament-containing adhesive mass.

The compounding amount of the total medicament component (glycyrrhetinic acid and any other

3

EP 0 156 565 B1

medical effect component or components) is e.g. 1.5 to 30%, preferably 3 to 20%, by weight of the total adhesive mass.

As the plaster base, one containing rubber compound adhesive plaster base, tackifying agent, and softener as the necessary components, or one containing acrylic resin as the necessary component,is used.

As the rubber compound adhesive plaster base, there are natural rubber and synthetic rubbers such as styrene-butadiene copolymer elastomer, styrene-isoprene-styrene copolymer elastomer, silicone rubber, etc. As the tackifying agent or component, there are petroleum resins such as Quintons (registered trade name, aliphatic petroleum resin, made by Nihon Zeon K. K.), Alcone (registered trade name, alicyclic petroleum resin, made by Arakawa Chemical Industries Co., Ltd.), etc., rosin, hydrogenated rosin, ester gum etc. As the softener there are polybutene, liquid paraffin, higher fatty acid esterssuch as isopropyl myristate, etc., silicone oils, vegetable oils etc.

Optional additives in the adhesive mass of the plasters of this invention include, for example, anti-oxidants such as butylated hydroxyanisole, guaiacol esters, butylated hydroxytoluene (BHT), nordihydroguaiaretic acid, etc.; antiseptics such as thymol, etc.; and diethylene glycol monoethyl ether as a solubilizer.

Polybutene is itself tacky and hence functions as a tackifying component but can be used as a softener since it softens a rubber component without the need for other softener.

When an acrylic resin such as an acrylic acid-acrylic acid ester series polymer, for example, Primal$^R$ N = 580 and ASE60 (trade names, made by Acryl Chemical Co., Ltd.), etc., is used as the base, a suitable plaster of this invention can be produced without using the above-described rubber compound adhesive plaster base, tackifying agent, and softener. In this case, the acrylic resin can be uniformly kneaded with the medicament solution and, if desired, the above-described base component to form a madicament-containing adhesive mass.

For producing the medicament-containing adhesive mass a solvent, hot melts, or emulsion method, etc., is usually used.

In one solvent method, a solution of glycyrrhetinic acid in above-described specific solvent is dissolved in (mixed with) further organic solvent and is mixed with a solution of above-described base in further organic solvent and the mixture is uniformly kneaded. As the further solvent(s) for dissolving (mixing with) the solution of the glycyrrhetinic acid and dissolving the base, an organic solvent which has compatibility with the base and does not deposit crystals when mixed with the solution of the glycyrrhetinic acid is selected. Examples of such an organic solvent are toluene, carbon tetrachloride, chloroform, methylene chloride, etc.

In one emulsion method, a method of converting the whole medicament-containing adhesive mass into an emulsion is employed and the emulsion is prepared using a latex emulsion of natural rubber and/or synthetic rubber; a latex emulsion having a rubber content of about 40 to 80% is suitably used.

When using rubber compound adhesive plaster base, tackifying agent, and softener as the base, it is advantageous for the compounding amount of the rubber compound adhesive plaster base to be about 15 to 60% by weight of the total amount of the medicament-containing adhesive mass, that of the tackifying agent about 15 to 60% by weight, and that of the softener 5 to 30% by weight.

Preferably the total amount of base(3) is 75 to 98.5%, particularly 80 to 97%,by weight of the total medicament-containing adhesive mass.

When using acrylic resin as the base, the compounding amount thereof is e.g. 65 to 98.5%, preferably 70 to 97%,by weight of the total medicament-containing adhesive mass.

When using the base in the form of emulsion, it is appropriate for the compounding amount of the solid component to be within the above ranges.

For production of the medicament-containing adhesive mass, the order of adding the medicament(1), any other medical effect component, the solvent, any additive, and the base may be varied as appropriate, and heating or ultrasonic treatment may be applied for accelerating dissolution in the solvent.

The medicament-containing adhesive mass thus prepared maybe spread on a support or backing and a release film applied on the adhesive mass layer; or the medicament-containing adhesive mass may be spread on a release film and a support or a backing applied thereon. When producing the adhesive medicament mass by the above-described solvent or emulsion method the further sclvent may be evaporated off, or water removed by drying,after spreading the adhesive mass,

As the support or backing, a material usually used in the field of this art, such as plastics film, flannel, unwoven fabric, etc., can be used.

As the release film, a suitably treated plastics film is usually used.

The plaster thus obtained can be fabricated to appropriate form according to the disease and location to be treated, and the various symptoms. For example, for dermatitis neurotica such as insect bite, etc., a

4

patch which is handy to carry and easily applicable is most preferred.

The present invention provides for the first time antipruritic plasters containing glycyrrhetinic acids as an essential medicinal component; these plasters can exhibit an adequate and durable medical effect without depositing crystals of glycyrrhetinic acid and other medicinal component.

Furthermore, the antipruritic plaster of this invention can be advantageous for concentrated treatment of a limited location; it need have no unpleasant smell, and does not stain clothes as do ordinary, sticky ointments.

When the plaster is in circular patch form, it is very handy to carry and very advantageous for application to insect bites, etc.

Tests on antipruritic plasters of this invention and the results thereof are shown below.

Test for comparing the effects of a product of this invention and of commercially available antipruritic agent for mosquito bite:

1. Test Procedure A (Determination test by medical doctor)

A tube for blood-sucking containing male imagos of Aedes aegypti in a blood-sucking period after the 2nd generation bred by hatching from the ovum is applied to the inner side of upper arms of 32 male and female healthy adults;

the test is applied simultaneously to three such portions of each arm. After blood-sucking for 10 minutes, leaving one portion untreated, two bite portions are treated by applying thereto a commercially available anti-pruritic agent (Muhi[R], made by Ikeda Mohando K. K., a solution for sticky coating having diphenhydramine as main anti-pruritic agent)and a circular plaster (60-100 $\mu$m in thickness of the medicament-containing adhesive mass) prepared according to Example 8 of this invention, wherein the combination of the medicaments and the compounding ratio of the medicaments are changed to some extent in the scope of this invention (the medicaments of the plaster of this invention are a combination of glycyrrhetinic acid, diphenhydramine, crotamiton, and isopropylmethylphenol).

The effect on itching is recorded based on the subjective symptom of the testees, at definite time intervals (0.5, 2, 4, 8, and 24 hours), and compared with the effect of the commercially available antipruritic agent.

The effect for each symptom is evaluated as follows according to the subjective symptoms of the testees.

3 --- Very itchy (Itch is too severe and they cannot concentrate on their work)
2 --- Considerably itchy (Itch is strong and they cannot concentrate on their work although they can work)
1 --- A little itchy (Itch not a hindrance to work)
0 --- Not itchy

The general determination is performed by ranking from the effect determination for each symptom (in addition, a same effect is in a same rank) and the effect is compared by the percentage of the number of the testees in the 1st rank of the determination results.

Test Result:

| | |
|---|---|
| Product of this invention | 53.8% |
| Commercially available product | 20.5% |
| Untreated | 5.1% |
| No difference | 20.8% |

2. Test Procedure B (panel test):

To 20 volunteers bitten by insects during the period from August to the middle of September, was applied a sensitivity test for determining the effect of the product of this invention and comparing it with that of commercially available products. Answers were obtained from 16 volunteers.

The determination of the effects was evaluated in three stages of (1) quickly effective, (2) effective, and (3) not effective against itching and (1) suppressing well, (2) suppressing, and (3) not suppressing swelling based on the subjective symptom of the testees.

The comparison with the commercially available products was made by the feelings of the testees on the relative effects of the product of this invention and the conventional commercially available product.

Test Result:

The results are shown by the percentage of the testees giving each evaluation:

(1) Effect of the Product of Invention:

(a) Itching
| | |
|---|---|
| Quickly effective | 56.2% |
| Effective | 43.8% |
| Not effective | 0 |
| No answer | 0 |

(b) Swelling
| | |
|---|---|
| Suppressing well | 56.2% |
| Suppressing | 12.5% |
| Not suppressing | 6.3% |
| No answer | 25.0% |

(2) Comparison with Commercially Available Product:

The commercially available products used and the percentage of the number of testees are as follows.
Muhi® (same as Test Procedure A) 45%
Kinkan® (trade name, made by Kinkando K. K., antipruritic effective component: aqueous ammonia, solution) 20%
Una Kowa® (trade name, made by Kowa K. K., antipruritic effective component: diphenhydramine hydrochloride, solution) 10%
Makiron® (trade name, made by Yamanouchi Pharmaceutical Co., Ltd., solution containing chlorpheniramine maleate as the antipruritic effective component) 25%

Comparison Result:

| | |
|---|---|
| Product of this invention | 60% |
| Commercially available product | 0 |
| Unable to evaluate | 40% |

Example 1

To sufficient amount of toluene were added 8.8 g of polybutene, 47.5 g of a petroleum resin (Quintons® U-185, trade name, made by Nihon Zeon K. K.), and 31.7 g of a styrene-isoprene-styrene copolymer resin (Cariflex® TR-1107, trade name, made by Shell Kagaku Co., Ltd.) and they were dissolved therein under heating; after cooling, a solution of 2.0 g of glycyrrhetinic acid and 2.0 g of diphenhydramine in 8.0 g of benzyl alcohol was uniformly mixed with the above solution. The mixture was coated on a release liner and after evaporating off toluene, a polyvinyl chloride film was applied thereto.

Examples 2 to 7

Plasters having the following adhesive medicament masses were prepared as in Example 1, using toluene as the further solvent which is evaporated after the coating onto the liner.

Example 2

6

| Glycyrrhizic acid | 1.0 g |
| Chlorpheniramine | 1.0 g |
| N-Methyl-2-pyrrolidone | 9.0 g |
| SIS* (Cariflex® TR-1107) | 32.0 h |
| Petroleum resin (Quintons® TR-1107) | 48.1 g |
| Polybutene | 8.9 g |
| | Total 100.0 g |

Example 3

| Dipotassium glycyrrhizinate | 0.5 g |
| Crotamiton | 10.0 g |
| Propylene glycol | 2.5 g |
| SIS* (Cariflex® TR-1107) | 31.3 g |
| Petroleum resin (Quintons^R U-185) | 47.0 g |
| Polybutene | 8.7 g |
| | Total 100.0 g |

Example 4

| Glycyrrhetinic acid | 1.0 g |
| Crotamiton | 5.0 g |
| Diphenhydramine | 1.0 g |
| Lauric acid diethanolamide | 5.0 g |
| SIS* (Cariflex® TR-1107) | 29.9 g |
| Petroleum resin (Quintons® U-185) | 49.3 g |
| Polybutene | 8.8 g |
| | Total 100.0 g |

Example 5

| Glycyrrhizic acid | 0.5 g |
| Crotamiton | 5.0 g |
| Chlorpheniramine maleate | 0.5 g |
| Benzyl alcohol | 5.0 g |

| SIS* (Cariflex® TR-1107) | 35.2 g |
| Petroleum resin (Quintons® U-185) | 44.8 g |
| Polybutene | 9.0 g |
| Total | 100.0 g |

* In the above Examples, SIS means a styrene-isoprene-styrene teleblock copolymer resin, made by Shell Kagaku Co., Ltd.

Example 6

In 15.0 g of crotamiton were dissolved 1.5 g of glycyrrhizic acid and 0.1 g of an antioxidant, butylated hydroxytoluene (BHT) and after uniformly mixing the solution with 151.6 g of an acrylic resin emulsion (Primal® N-580-S, trade name, made by Acryl Chemical Co., Ltd., solid component 54.5-55.5%), the mixture was coated on a release liner followed by drying. Thereafter, a polyvinyl chloride film was applied thereto.

Examples 7 and 8

Plasters having the following adhesive medicament masses were prepared in the manner of Example 7.

Example 7

| Glycyrrhetinic acid | 1.0 g |
| Crotamiton | 6.0 g |
| Chlorpheniramine | 1.0 g |
| Isopropylmethylphenol | 1.0 g |
| BHT | 0.1 g |

Acrylic resin emulsion (Primal®
N-580-S)                                       165.2 g

                       Total           174.3 g

                    (100.0g as solid component)


Example 8

Glycyrrhetinic acid                      0.5 g

Crotamiton                              10.0 g

Diphenhydramine                         1.0 g

BHT                                     0.1 g

Acrylic resin emulsion
(Primal$^R$ N-580-S)                     160.6 g

                    Total              172.2 g

                    (99.9 g as solid component)


Reference Example

A mixture of 18.8 g of polybutene, 35.2 g of a petroleum resin (Quintons® U-185, trade name, made by Nihon Zeon K. K.) and 4.9 g of liquid paraffin was heated to 100 to 120°C to form a solution and after allowing the solution to cool to a temperature of 50-60°c, the solution was uniformly mixed with a solution of 1.0 g glycyrrhizic acid and 0.1 g of BHT in 5.0 g of N-methyl-2-pyrrolidone. The mixture thus formed was further uniformly mixed with 25.5 g (60% solid component) of a styrene-butadiene copolymer latex and 19.5 g (60% solid component) of a natural rubber latex; the resultant mixture was coated on a release liner followed by drying and a polyvinyl chloride film was applied thereto.

Examples 10 to 13

Plasters with the following adhesive medicament masses were prepared in the manner described in the Reference Example.

Example 10

| Glycyrrhizic acid | 0.5 g |
|---|---|
| Diphenhydramine | 2.0 g |
| Lauric acid diethanolamide | 2.0 g |
| BHT | 0.1 g |
| Petroleum resin (Quintons® U-185) | 32.5 g |
| Polybutene | 20.0 g |
| Liquid paraffin | 4.9 g |
| Styrene-butadiene copolymer latex | 23.2 g (60% solid component) |
| Natural rubber latex | 14.8 g (60% solid component) |
| Total | 100.0 g |
| | (84.8 g as solid component) |

Example 11

| Glycyrrhetinic acid | 1.0 g |
|---|---|
| Diphenhydramine | 1.0 g |
| Crotamiton | 6.0 g |
| Isopropylmethylphenol | 1.0 g |
| BHT | 0.1 g |
| Petroleum resin (Quintons® U-185) | 40.3 g |
| Polybutene | 15.7 g |
| Liquid paraffin | 4.9 g |
| Styrene butadiene copolymer latex | 29.0 g |

10

(60% solid component)

Natural rubber latex                    21.0 g
                              (60% solid component)

                    Total         120.0 g

              (100.0 g as solid component)


Example 12

Glycyrrhetinic acid                  1.0 g

Diphenhydramine                      2.0 g

Isopropylmethylphenol                0.5 g

N-Methyl-2-pyrrolidone               2.0 g

Diethylene glycol monoethyl
ether                                2.0 g

BHT                                  0.1 g

Petroleum resin (Quintons®
U-185)                              35.2 g

Polybutene                          17.3 g

Liquid paraffin                      4.9 g

Styrene-butadiene copolymer         26.0 g
latex                       (60% solid component)

Natural rubber latex                19.0
                              (60% solid component)

                    Total         110.0 g

              (92.0 g as solid component)


Example 13

| | |
|---|---|
| Glycyrrhetinic acid | 0.5 g |
| Diphenhydramine | 2.0 g |
| Isopropylmethylphenol | 0.5 g |
| Isopropyl myristate | 4.0 g |
| BHT | 0.1 g |
| Petroleum resin (Quintons® U-185) | 34.0 g |
| Styrene-butadiene copolymer latex | 23.7 g (60% solid component) |
| Natural rubber latex | 16.3 g (60% solid component) |

Total 100.0 g

(87.0 g as solid

component)

## Claims

1. An antipruritic plaster comprising a backing having formed on one surface thereof a pressure-sensitive adhesive medicament layer containing
   (1) at least one medicament selected from glycyrrhetinic acid glycyrrhizin, and salts of glycyrrhizin;
   (2) at least one solvent for (1) selected from benzyl alcohol, phenethyl alcohol, diphenhydramine, chlorpheniramine and crotamiton; and
   (3) a base comprising
   [a] at least one rubber compound adhesive plaster base selected from natural rubber, styrene-butadiene copolymer elastomers, and styrene-isoprene-styrene copolymer elastomers; at least one tackifying agent selected from petroleum resin, rosin, hydrogenated rosin, polybutene, and ester gum; and at least one softener selected from polybutene, liquid paraffin, higher fatty acid esters, silicone oils, and vegetable oils or
   [b] acrylic resin.

2. A plaster according to claim 1 wherein the adhesive medicament layer contains 0.1 to 10% by weight of component (1), 3 to 20% by weight of component (2), together with 75 to 98.5% by weight of component (3) [a] or 65 to 98.5% by weight of component (3) [b].

3. A plaster according to claim 1 wherein the adhesive medicament layer contains 0.2 to 4% by weight of component (1), 5 to 15% by weight of component (2), and 70 to 97% by weight of component (3) [b].

4. A plaster according to claim 1 wherein the adhesive medicament layer contains glycyrrhetinic acid, diphenhydramine, crotamiton, and an acrylic resin base and optionally at least one of antibacterial, antioxidant and odorant agents.

12

5.  An adhesive medicament mass for use in a plaster, the mass containing
    (1) at least one medicament selected from glycyrrhetinic acid glycyrrhizin, and salts of glycyrrhizin;
    (2) at least one solvent for (1) selected from benzyl alcohol, phenethyl alcohol, diphenhydramine, chlorpheniramine and crotamiton; and
    (3) a base comprising
    [a] at least one rubber compound adhesive plaster base selected from natural rubber, styrene-butadiene copolymer elastomers, and styrene-isoprene-styrene copolymer elastomers; at least one tackifying agent selected from petroleum resin, rosin, hydrogenated rosin, polybutene, and ester gum; and at least one softener selected from polybutene, liquid paraffin, higher fatty acid esters, silicone oils, and vegetable oils or
    [b] acrylic resin.

**Revendications**

1.  Un pansement antipruritique comprenant un support sur une face duquel est formée une couche de médicament adhésive sensible à la pression contenant :
    (1) au moins un médicament choisi parmi l'acide glycyrrhétinique, la glycyrrihizine et les sels de la glycyrrhizine;
    (2) au moins un solvant pour (1) choisi parmi l'alcool benzylique, l'alcool phénéthylique, la diphénhydramine, la chlorphéniramine et le crotamiton; et
    (3) une base comprenant
    [a] au moins une base de pansement adhésif en composé de caoutchouc choisie parmi le caoutchouc naturel, les élastomères de copolymères styrène-butadiène, et les élastomères de copolymères styrène-isoprène-styrène; au moins un agent d'adhésivité choisi parmi la résine de pétrole, la colophane, la colophane hydrogénée, le polybutène et l'ester de colophane; et au moins un plastifiant choisi parmi le polybutène, la paraffine liquide, les esters d'acides gras supérieurs, les huiles de silicone, et les huiles végétales ou
    [b] une résine acrylique.

2.  Un pansement selon la revendication 1, dans lequel la couche de médicament adhésive contient de 0,1 à 10% en poids du composant (1), de 3 à 20% en poids du composant (2), en même temps que de 75 à 98,5% en poids du composant (3) [a] ou de 65 à 98,5% en poids du composant (3) [b].

3.  Un pansement selon la revendication 1, dans lequel la couche de médicament adhésive contient de 0,2 à 4% en poids du composant (1), de 5 à 15% en poids du composant (2), et de 70 à 97% en poids du composant (3) [b].

4.  Un pansement selon la revendication 1, dans lequel la couche de médicament adhésive contient de l'acide glycyrrhétinique, de la diphénhydramine, du crotamiton, et une base en résine acrylique, et éventuellement au moins un agent choisi parmi les agents antibactériens, antioxydants et odorants.

5.  Une masse de médicament adhésive destinée à être utilisée dans un pansement, la masse contenant
    (1) au moins un médicament choisi parmi l'acide glycyrrhétinique, la glycyrrhizine et les sels de la glycyrrhizine;
    (2) au moins un solvant pour (1) choisi parmi l'alcool benzylique, l'alcool phénétylique, la diphénhydramine, la chlorphéniramine et le crotamiton; et
    (3) une base comprenant
    [a] au moins une base de pansement adhésif en composé de caoutchouc choisie parmi le caoutchouc naturel, les élastomères de copolymères styrène-butadiène, et les élastomères de copolymères styrène-isoprène-styrène; au moins un agent d'adhésivité choisi parmi la résine de pétrole, la colophane, la colophane hydrogénée, le polybutène et l'ester de colophane; et au moins un plastifiant choisi parmi le polybutène, la paraffine liquide, les esters d'acides gras supérieurs, les huiles de silicone, et les huiles végétales ou
    [b] une résine acrylique.

**Patentansprüche**

1.  Antipruritisches Pflaster, umfassend eine Unterlage, auf der auf einer Oberfläche eine druckempfindliche Klebemittel/Arzneimittel-Schicht aufgebracht ist, enthaltend

(1) mindestens ein Arzneimittel, ausgewählt aus Glycyrrhetinsäure, Glycyrrhizin und Salzen von Glycyrrhizin;

(2) mindestens ein Lösungsmittel für (1), ausgewählt aus Benzylalkohol, Phenethylalkohol, Diphenhydramin, Chlorpheniramin und Crotamiton; und

(3) eine Basis, umfassend

(a) mindestens eine Pflasterbasis aus einer Kautschukverbindung, ausgewählt aus natürlichem Kautschuk, Styrol-Butadien-Copolymer-Elastomere und Styrol-Isopren-Styrol-Copolymer-Elastomere, mindestens ein klebrig-machendes Mittel, ausgewählt aus Erdölharz, Harzseifen, hydrierten Harzseifen, Polybuten und Estergummen, und mindestens einen Weichmacher, ausgewählt aus Polybuten, flüssigem Paraffin, höheren Fettsäureestern, Siliconölen und pflanzlichen Ölen, oder

(b) Acrylharz.

2. Pflaster nach Anspruch 1, wobei die Klebemittel/Arzneimittel-Schicht 0,1 bis 10 Gew.-% der Komponente (1), 3 bis 30 Gew.-% der Komponente (2) zusammen mit 75 bis 98,5 Gew.-% der Komponente (3) (a) oder 65 bis 98,5 Gew.-% der Komponente (3) (b) enthält.

3. Pflaster nach Anspruch 1, wobei die Klebemittel/Arzneimittel-Schicht 0,2 bis 4 Gew.-% Komponente (1), 5 bis 15 Gew.-% Komponente (2) und 70 bis 97 Gew.-% Komponente (3) (b) enthält.

4. Pflaster nach Anspruch 1, wobei die Klebemittel/Arzneimittelschicht Glycyrrhetinsäure, Diphenhydramin, Crotamiton und eine Acrylharzbasis und gegebenenfalls mindestens ein antibakterielles Mittel, Antioxidans oder einen Duftstoff enthält.

5. Klebende Arzneimittelmasse zur Verwendung in einem Pflaster, enthaltend

(1) mindestens ein Arzneimittel, ausgewählt aus Glycyrrhetinsäure, Glycyrrhizin und Salzen von Glycyrrhizin;

(2) mindestens ein Lösungsmittel für (1), ausgewählt aus Benzylalkohol, Phenethylalkohol, Diphenhydramin, Chlorpheniramin und Crotamiton; und

(3) eine Basis, umfassend

(a) mindestens eine Pflasterbasis aus einer Kautschukverbindung, ausgewählt aus natürlichem Kautschuk, Styrol-Butadien-Copolymer-Elastomere und Styrol-Isopren-Styrol-Copolymer-Elastomere, mindestens ein klebrig-machendes Mittel, ausgewählt aus Erdölharz, Harzseifen, hydrierten Harzseifen, Polybuten und Estergummen, und mindestens einen Weichmacher, ausgewählt aus Polybuten, flüssigem Paraffin, höheren Fettsäureestern, Siliconölen und pflanzlichen Ölen, oder

(b) Acrylharz.